# EUROPEAN PATENT APPLICATION

(11) **EP 2 526 947 A1**
(43) Date of publication of application: **28.11.2012**
(21) Application number: 11734273.3
(22) Date of filing: 19.01.2011
(51) Int. Cl.: A61K 31/472, C07D 217/04, A61P 25/28

(54) **USE OF AT LEAST ONE ISOQUINOLINE COMPOUND OF FORMULA I, PHARMACEUTICAL COMPOSITION FOR TREATING OR PREVENTING NEURODEGENERATIVE DISEASES AND METHOD FOR TREATING OR PREVENTING NEURODEGENERATIVE DISEASES**

(30) Priority: 20.01.2010 BR PI1001988
(71) Applicant: Pianowski, Luiz Francisco, 12917-025 Bragança Paulista - SP (BR); Calixto, João Batista, 88037-120 Florianópolis - SC (BR); Glinski, Jan A., Danbury, CT 06810 (US)
(72) Inventor: Pianowski, Luiz Francisco, 12917-025 Bragança Paulista - SP (BR); Calixto, João Batista, 88037-120 Florianópolis - SC (BR); Glinski, Jan A., Danbury, CT 06810 (US)
(74) Representative: KATZAROV S.A.
(86) International application number: PCT/BR2011/000022
(87) International publication number: WO 2011/088536

(57) **Abstract**

The present invention concern the use of isoquinoline alkaloid compounds of formula I in the preparation of pharmaceutical compositions useful in the treatment of neurodegenerative diseases, particularly Alzheimer's disease, as well as pharmaceutical composition comprising said compounds and method of treatment of neurodegenerative diseases. 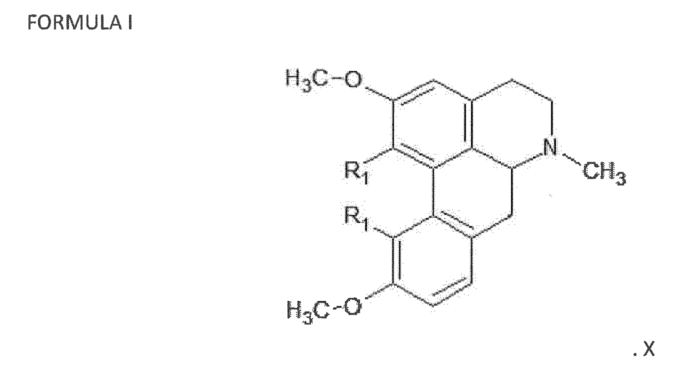 wherein R₁ is -OH or -OCH₃, and X is non-existent, HCl or HBr, including their isomers,

## Description

### Field of the invention

The present invention concerns the use of specific isoquinoline compounds for the preparation of pharmaceutical compositions useful in the treatment of neurodegenerative diseases, particularly Alzheimer's disease, as well as pharmaceutical compositions comprising said compounds and methods of treating neurodegenerative diseases.

### Background of the invention

With the unprecedented ageing of the world population, dementia has gained the status of a serious public health problem, once its prevalence tends to increase after the age of 65, and double every five years thereafter. According to the World Health Organization, it is estimated that 37 million people around the world suffer from dementia, among which 18 million have Alzheimer's disease Mount & Downton : Nat Med, 12:780-784, 2006).

Dementia is a general term employed to designate several neurodegenerative diseases, that are characterized when a person who had a normal intellectual development loses or decreases the cognitive capacity, partial or totally, permanently, momentaneously or occasionally. This overall decline in cognition results in progressive functional, social and professional loss.

Dementias include Alzheimer's disease, vascular dementia, Lewy body dementia, frontotemporal dementia, Korsakoff's dementia, among others.

Dementias may have different origins. Within the domains of dementia, the most common is Alzheimer's disease. The histopathological basis of the disease was described by the first time by the German neuropathologist Alois Alzheimer, who identified senile plaques (aggregates of beta-amyloid protein) and neurofibrillary tangles (associated to mutations and consequent hyperphosphorylation of the tau protein in the interior of the cytoskeleton microtubules of the neurons). Those two pathological findings, in an elderly patient with severe neuron-cognitive disorders, in the absence of evident impairment or intravascular lesion, allowed the characterization of this clinical condition as distinct from other organic pathologies of the brain.

The Alzheimer's settles when the processing of certain proteins of the central nervous system begins to go wrong. Then fragments of toxic, improperly cut proteins begin to appear within the neurons and in the spaces that exist among them. As a consequence of that toxicity, a progressive loss of neurons occur in certain regions of the brain, as in the hippocampus, that controls language and reasoning, memory, sensorial stimulation recognition and abstract thinking.

The beta-amyloid protein deposits in plaques, also known as neuritic plaques, of a spherical aspect, with a dense accumulation of beta-amyloid Aβ1 protein in the center, surrounded by a ring comprised of abnormal neuron particles. Those plaques cause the destruction of neurons by creating a chronic inflammatory process in the affected regions, interfering with the regulation of calcium which is essential for the conduction of nervous stimuli, and augmenting the production of free radicals, toxic for the nervous cells.

The Alzheimer's disease usually evolves in a slow and inexorable manner. From the diagnostic, the average survival rate is from 8 to 10 years. Patients will present alterations in memory, personality, visual and spatial abilities, impairment in speech, in performing simple tasks and in movement coordination, restlessness and insomnia, resistance to performing everyday tasks, urinary and fecal incontinence, eating difficulties, progressive motor impairment, restriction to bed, mutism, swallowing pain, intercurrent infections, among other symptoms.

Presently, the first line treatment offered to dementias is based on cholinesterase-inhibiting medications, - for instance, donepezil, rivastigmine or galantamine - which offer some help concerning the cognitive loss characteristic of dementias, but providing limited improvement. The present treatment aims to give comfort to the patient, retarding as much as possible the evolution of the disease.

Therefore, there is a need for alternatives that may safely and effectively prevent or treat the neurodegenerative diseases, and not only minimizing their symptoms.

### DESCRIPTION OF FIGURES

The present invention is illustrated by the following attached figures:
Figure 1 shows a comparative graph of escape latency profile in animals that received vehicle only (control), p-amyloid ₁₋₄₀ (Aβ₁₋₄₀), and β-amyloid ₄₀₋₁ (Aβ₄₀₋₁).
Figure 2 shows a comparative graph of percentage of time spent in the right quadrant in animals that received vehicle only (controls β-amyloid ₁₋₄₀ (Aβ₁₋₄₀), or β-amyloid 40-1 (Aβ₄₀₋₁).
Figure 3 shows a comparative graph of the training test results in the platform performed with and without the use of the isoquinoline alkaloid compounds of formula I according to the invention.
Figure 4 shows a comparative graph of training tests results without platform with respect of time in the proper quadrant, performed with an without the use of the isoquinoline alkaloid compounds of formula I according to the invention.
Figure 5 shows a comparative graph of training tests results without platform, with respect to the distance travelled (meters), performed with and without the use of the isoquinoline alkaloid compounds of formula I according to the invention.
Figure 6 shows a comparative graph of training tests results without platform, with respect of average speed (meters per second), performed with and without the use of the isoquinoline alkaloid compounds of formula I according to the invention.

### DESCRIPTION OF THE INVENTION

The present invention has as an object the use of at least one isoquinoline compound of the formula I below in the preparation of pharmaceutical compositions useful in the treatment of neurodegenerative diseases. 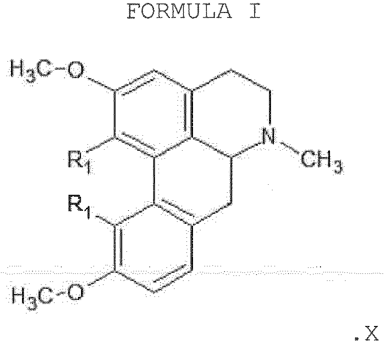 wherein R₁ is -OH or -OCH₃, and X is non-existent, HCl or HBr, including their isomers.

The inventors observed that the compounds of formula I, for instance corydine or isocorydine, act directly upon the beta-amyloid plaques, reducing their deposits. Furthermore, said compounds stimulate action in other areas of the brain, what results in efficacy in the treatment as a whole.

Neurodegenerative diseases according to the present invention include those related to the action of beta-amyloid plaques, particularly Alzheimer's disease, vascular dementia, Lewy body dementia, frontotemporal dementia, Korsakoff dementia, more particularly Alzheimer's disease. In a further aspect, the present invention relates to pharmaceutical compositions useful in treating or preventing neurodegenerative diseases comprising, as active, at least one isoquinoline alkaloid compound of formula I and pharmaceutically acceptable excipients.

The present invention also concerns mixtures of at least two isoquinoline compounds, for instance, mixtures of corydine and isocorydine, particularly in proportions between 1:100 to 100:1.

The pharmaceutically acceptable excipients according to the invention, without any limitation, may be selected among those cited in the following references: Remington's Pharmaceutical Sciences, Mack Publishing, European or Brazilian Pharmacopeias, and new excipients to be developed.

Pharmaceutical compositions according to the invention are formulated for oral administration, as solids, liquids or semi-solids, for instance tablets, capsules pills, powder, granules, pellets, suspensions, emulsions, dispersions or any other form known in the art.

In another aspect, the present invention concerns a method to treat or prevent neurodegenerative diseases that comprise administering to a patient in need a pharmaceutically efficacious amount of at least one isoquinoline alkaloid compound of formula I.

Adequate dosages according to the present invention vary from 0.01 to 1000 mg/kg of patient weight of at least one isoquinoline alkaloid compound of formula I, according to the needs of the patient.

The following examples aim to illustrate aspects of the present invention without having any limitative character.

### EXAMPLES

### Example 1 - Evaluation of effect in neurodegenerative disease - animal model of Alzheimer's disease induced by Aβ₁₋₄₀.

The test was performed in mice, by way of intracerebrovascular (ICV)injection, by hand, in the lateral ventricle, of phosphate-containing saline solution (PBS - phosphate buffered solution), β-amyloid ₁₋₄₀ (Aβ, 400 pmol) and β-amyloid ₄₀₋₁ (revAβ, 400 pmol), according to method described by Haley & McCormic (Br J Pharmacol, 12:12-15, 1957) and Laursen & Belknap (J Pharmacol Methods, 16:355-357, 1986), hereby incorporated by reference.

### Animal model of Alzheimer's disease induced by Aβ₁₋₄₀.

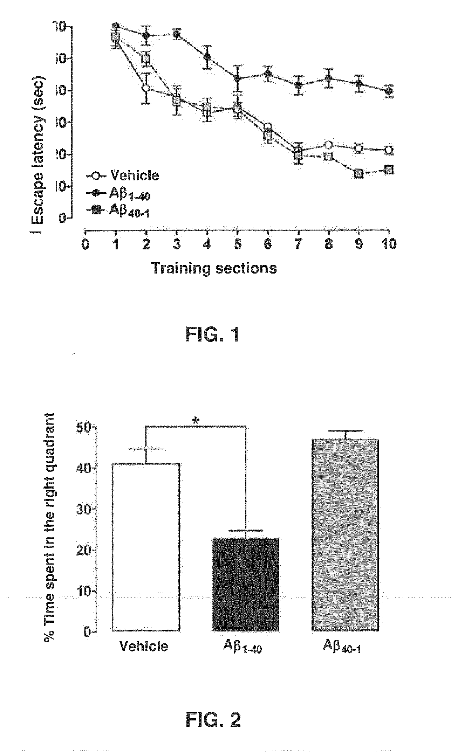

The graph shown in figure 1, according to Medeiros et al. (J Neurosci, 27:5394-5404, 2007), hereby also incorporated by reference, show that animals that received Aβ₁₋₄₀ or Aβ₄₀₋₁ present smaller escape latency than those who received only vehicle (control).

The graph shown in figure 2, also according to Medeiros et al., demonstrate that animals that received Aβ1-40 present less percentage of time spent in the correct quadrant with relation to those that received vehicle (control) or Aβ₄₀₋₁.

The results reveal that Aβ₁₋₄₀ interrupts the progress of learning and memory of the animals.

### Effect in the cognitive deficit induced by Aβ₁₋₄₀

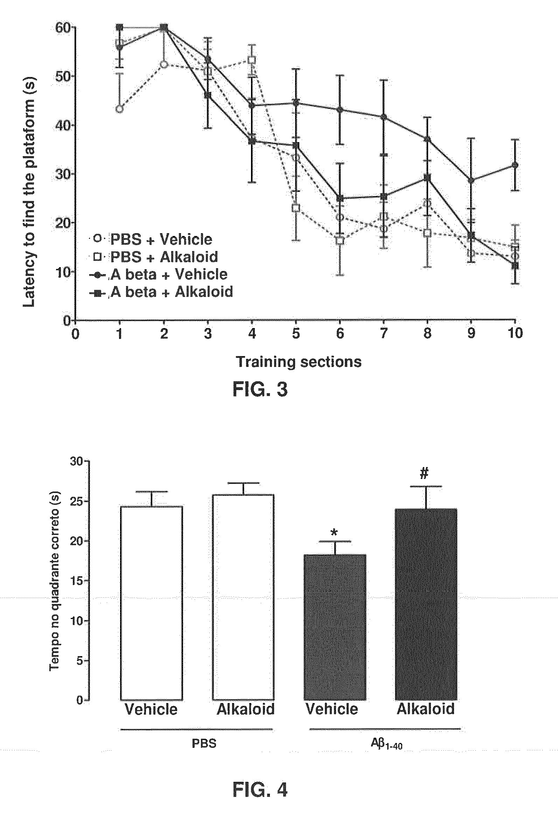

The graph in figure 3 shows the results of training tests in the platform performed with and without the use of the compounds according to the present invention (50 mg/kg, po (orally), once a day, from day 0 to day 6), after 7 days of treatment.

Animals were divided in four groups:
- group 1 received PBS and vehicle (control).
- group 2 received PBS and the compound according to the invention (1:1 mixture of corydine and isocorydine).
- group 3 received Aβ and vehicle
- group 4 received Aβ and the compound according to the invention.

The results reveal that the use of the compound of the invention significantly reduces the latency to find the platform in 10 training sessions.

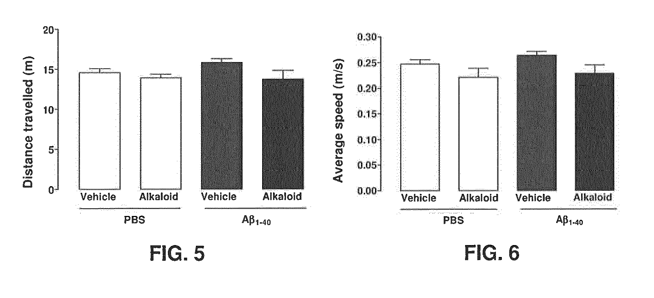

The graphs of figures 4 to 6 show the results of training tests without platform performed with and without the use of the compounds according to the present invention (50 mg/kg, po (orally), once a day, from day 0 to day 6), after 8 days of treatment.

Animals were divided in four groups:
- group 1 received PBS and vehicle (control).
- group 2 received PBS and the compound according to the invention (1:1 mixture of corydine and isocorydine).
- group 3 received Aβ and vehicle
- group 4 received Aβ and the compound according to the invention.

The results reveal that the animals that received the compound according to the invention show significantly better performance in all performed tests.

### Visual Behavioral Characterization of compound

The animals that received the compound of the invention (50 mg/kg, po) were evaluated during 24 hours.

It was verified that the animals presented response to the touch, to tail pressing, corneal reflex and corporal tonus. Not observed were cardiac or respiratory frequency increase, contortion, trembling, convulsions, straub signal, ptosis (lacrimation) or piloerection.

### RESULTS

According to the results of the tests performed it was proven that the compound of the invention is able to prevent cognitive damage induced by Aβ₁₋₄₀ in an atoxic dosage.

Therefore, the compounds according to the invention can be used in the preparation of medicaments aimed to the prevention and treatment of neurodegenerative diseases, particularly Alzheimer's disease.

It must be understood that the embodiments described herein are merely examples and that modifications are at the reach of a person skilled in the art. Consequently the present invention is not to be considered limited only to the embodiments described herein.

## Claims

1. Use of at least one isoquinoline compound of formula I 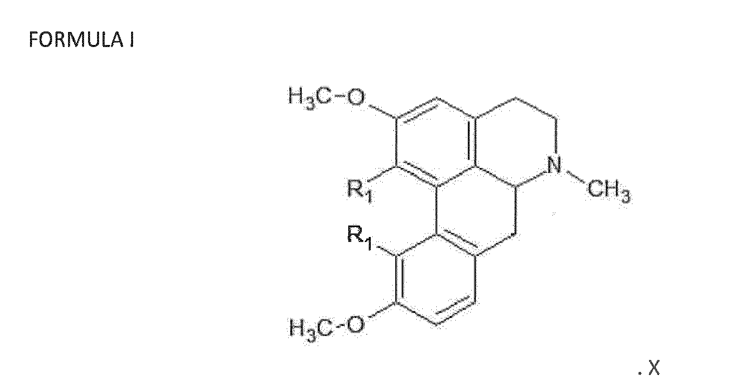 wherein R₁ is -OH or -OCH₃, and X is non-existent, HCl or HBr, including their isomers, in the preparation of pharmaceutical compositions useful in the treatment or prevention of neurodegenerative diseases.

2. Use, according to claim 1, wherein the compounds of formula I are chosen from corydine, isocorydine and their mixtures.

3. Use, according to one of claims 1 or 2, wherein the compounds are a moisture 1:100 to 100:1 of corydine and isocorydine.

4. Use according to claim 1, wherein the neurodegenerative diseases are one or more of Alzheimer's disease, vascular dementia, Lewy body dementia, frontotemporal dementia and Korsakoff's dementia.

5. Use according to claim 1, wherein the neurodegenerative diseases is Alzheimer's disease.

6. Pharmaceutical composition to treat or prevent neurodegenerative diseases comprising at least one isoquinoline compound according to one of claims 1 to 3, and pharmaceutically acceptable excipients.

7. Pharmaceutical composition according to claim 6 wherein said neurodegenerative diseases are one or more of Alzheimer's disease, vascular dementia, Lewy body dementia, frontotemporal dementia and Korsakoff's dementia.

8. Pharmaceutical composition according to claim 6 wherein said neurodegenerative diseases is Alzheimer's disease,

9. Method to treat or prevent neurodegenerative diseases comprising the administration to a patient in need of treatment at least one isoquinoline compound according to one of claims 1 to 3.

10. Method, according to claim 9, wherein the neurodegenerative diseases are one or more of Alzheimer's disease, vascular dementia, Lewy body dementia, frontotemporal dementia and Korsakoff's dementia.

11. Method according to claim 10 wherein the neurodegenerative diseases is Alzheimer's disease.

12. Method according to one of claims 9 to 11 comprising the administration of 0.01 to 1000 mg/kg of patient weight of at least one isoquinoline of formula I, one or more times a day.
